# EUROPÄISCHE PATENTSCHRIFT

(11) **EP 2 162 115 B1**
(45) Veröffentlichungstag und Bekanntmachung des Hinweises auf die Patenterteilung: **18.12.2013**
(21) Anmeldenummer: 08774421.5
(22) Anmeldetag: 27.06.2008
(51) Int. Cl.: A61K 8/44, A61K 8/60, A61Q 19/08, A61Q 19/00, A61Q 7/02

(54) **ADVANCED GLYCATION ENDPRODUCTS ALS WIRKSTOFFE**
ADVANCED GLYCATION END PRODUCTS AS ACTIVE INGREDIENTS
PRODUITS FINAUX DE LA GLYCOSYLATION AVANCÉE EN TANT QUE SUBSTANCES ACTIVES

(30) Priorität: 10.07.2007 DE 102007032393
(43) Veröffentlichungstag der Anmeldung: 17.03.2010
(73) Patentinhaber: Henkel AG & Co. KGaA, 40589 Düsseldorf (DE)
(72) Erfinder: HUCHEL, Ursula, 50670 Köln (DE); KROPF, Christian, 40724 Hilden (DE); WELSS, Thomas, 40591 Düsseldorf (DE); GIESEN, Melanie, 47608 Geldern (DE); BOCK, Andreas, 40591 Düsseldorf (DE)
(86) Internationale Anmeldenummer: PCT/EP2008/058255
(87) Internationale Veröffentlichungsnummer: WO 2009/007257

(56) Entgegenhaltungen:
- WO-A2-03/089601
- WO-A2-2005/051288
- DE-A1- 2 364 525
- DE-A1- 10 041 482
- DE-A1- 10 160 966
- US-B1- 6 218 435
- THOMAS HENLE: 'Efficient determination of individual Maillard compounds in heat-treated milk products by amino-acid analysis' INTERNATIONAL DAIRY JOURNAL, [Online] 01 Januar 1991, Seiten 125 - 135, XP055033133 Gefunden im Internet: <URL:http://ac.els-cdn.com/095869469190004R /1-s2.0-095869469190004R-main.pdf?_tid=30fe 52ac5998dc46e724b2727772361a&acdnat=1342613 696_06bfeec61cc13a0f9ed00260d9e170fa> [gefunden am 2012-07-18]
- JOSÉ A.B BAPTISTA ET AL: 'Indirect determination of Amadori compounds in milk-based products by HPLC/ELSD/UV as an index of protein deterioration' FOOD RESEARCH INTERNATIONAL Bd. 37, Nr. 8, 01 Januar 2004, Seiten 739 - 747, XP055033131 DOI: 10.1016/j.foodres.2004.02.006 ISSN: 0963-9969
- FRANZ LEDL: 'Formation of maltosine, a product of the Maillard reaction with a pyridone structure' ZEITSCHRIFT FÜR LEBENSMITTEL-UNTERSUCHUNG UND -FORSCHUNG, [Online] 01 Januar 1989, Seiten 207 - 211, XP055033149 Gefunden im Internet: <URL:http://www.springerlink.com/content/r6 k1306376q12564/fulltext.pdf> [gefunden am 2012-07-18]

## Beschreibung

Die vorliegende Erfindung betrifft die Verwendung einer kosmetischen Zusammensetzung enthaltend mindestens eine ausgewählte Verbindung aus der Gruppe der AGEs (Advanced Glycation Endproducts) und/oder deren Vorläufern als Haulbehandlungsmittel zur Reduktion des Haarwuchses und/oder zur Reduktion der Haardicke.

Die Offenlegungsschrift DE 10041482 offenbart sog. AGEs (Advanced Glycation Endproducts) als kosmetische wirkstoffe zur Induktion und Intensivierung von Bräunungsmechanismen der menschlichen Haut und die Verwendung einer oder mehrerer Substanzen aus der Gruppe der AGEs und/oder deren Vorläufern zur Steigerung der Melaninsynthese der Haut.
Die Offenlegungsschrift DE 10160966 enthält die Lehre zur Bereitstellung eines Haarfärbemittels enthaltend mindestens eine Verbindung aus der Gruppe der AGEs, deren synthetischen Vorstufen und/oder der Lipofuscine und die Verwendung eines solchen Haarfärbemittemittels zur Steigerung der Melaninsynthese in der Haarwurzel und der Einlagerung von Malanin im Haar.

Beiden Schriften ist gemeinsam, dass kosmetische Wirkstoffe aus der Gruppe der AGEs zur Steigerung der Melaninsynthese im Haar und/oder der Haut benutzt werden. Ein Hinweis auf andere oder weitergehende biologisch relevante Wirkungen der AGEs sind beiden Dokumenten nicht zu entnehmen.
US 6218435 B1 offenbart ein Verfahren zur Reduktion von menschlichem Haarwuchs, bei dem 2-Deoxyglucose oder 3-Deoxyglucose auf die Haut aufgetragen wird.
Überraschenderweise wurde nun gefunden, dass weitere ausgewählte Verbindungen aus der Gruppe der AGEs und/oder deren Vorläufern auch als Hautbehandlungsmittel zur Reduktion des Haarwuchses und/oder zur Reduktion der Haardicke verwendet werden können.

Gegenstand der vorliegenden Erfindung ist daher die Verwendung einer kosmetischen Zusammensetzung enthaltend mindestens eine Verbindung aus der Gruppe der AGEs und/oder deren Vorläufern, ausgewählt aus Verbindungen mit einer chemischen Struktur gemäß Formel 15 oder 15a, als Hautbehandlungsmittel zur Reduktion des Haarwuchses und/oder zur Reduktion der Haardicke.

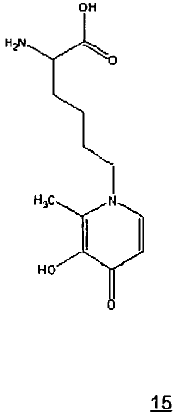

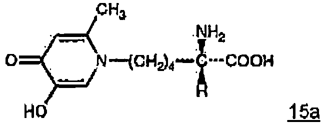

Ein weiterer Gegenstand der vorliegenden Erfindung ist die Verwendung von Maltosin als Anti-Haarwuchsmittel.

Gegenstand der vorliegenden Erfindung ist weiterhin die nicht-therapeutische Verwendung einer kosmetischen Zusammensetzung, enthaltend mindestens eine Verbindung aus der Gruppe der Lysin- basierten AGEs und/oder deren Vorläufern, enthaltend mindestens eine Verbindung aus der Gruppe der Lysin-basierten AGEs und/oder deren Voläufern, ausgewählt aus Verbindungen mit einer chemischen Struktur gemäß Formel 15 oder 15a, zur Hautbehandlung.

In einer bevorzugten Ausführungsform enthält die oben genannte kosmetische Zusammensetzung als weitere Komponente einen Emutgator bzw. ein Tensid, das bevorzugt aus der Gruppe der anionischen, kationischen, amphoteren und/oder nicht-ionischen Tenside bzw Emulgatoren oder aus deren Mischungen ausgewählt wird.
Besonders bevorzugt enthält die kosmetische Zusammensetzung zumindest eine weitere Komponente ausgewählt aus der Gruppe der Kosmetischen Wirkstoffe, Vitamine, Proteinhydrolysate, filmbildende Substanzen, UV-Filtersubstanzen, Duftstoffe, Kondilioniermittelwirkstoffe, Verdickungsmittel, Konservierungsmittel, Antioxidantien, Färbemittel, Puffermittel. Sequestrierungsprodukte, Treibmittel, Reduktionsmittel, Antischuppenwirkstoffe, Tanning-Mittel. Feuchthaltemittel der Haut und/oder Glanzmittel.

Erfindungsgemäß sind unter dem augemein verwendeten Begriff "Haut" die Haut selbst, die Schleimhaut sowie die Hautanhangsgebilde, sofern sie lebende Zellen umfassen, insbesondere Haarfollikel, Haarwurzel, Haarzwiebel, das ventrale Epithel des Nagelbetts (Lectulus) sowie Talgdrüsen und Schweißdrüsen, zu verstehen. Bevorzugt Im Sinne der Erfindung ist unter dem allgemein verwendeten Begriff "Haut" die "menschliche Haut" zu verstehen.

Die Bezeichnung AGE kommt vom englischen Begriff "Advanced Glycation Endproducts". AGEs im Sinne der vorliegenden Erfindung sind beispielsweise erhältlich gemäß der folgenden (beispielhaften) Reaktionsgleichung:

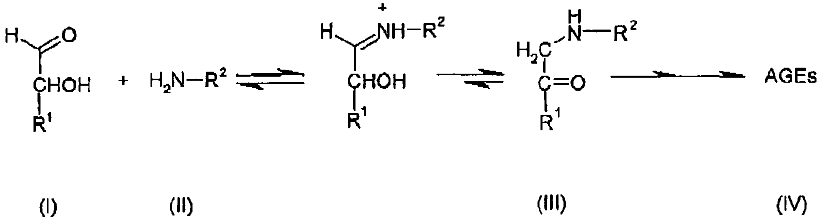

Die dargestellten Strukturformeln sollen die Erfindung selbstverstandlich nicht auf bestimmte Isomere der erfindungsgemaßen Substanzen beschränken. Vielmehr sind auch nicht dargestellte Isomere bzw. Isomerengemische vorteilhaft im Sinne der vorliegenden Erfindung.

Ein bevorzugtes Lösungsmittel für die Darstellung der erfindungsgemäßen AGEs ist Wasser, das vorzugsweise mit einem Phosphatgemisch als Puffer auf einen pH-Wert zwischen 3,0 und 10,0. vorzugsweise auf 3,0, 4,0, 5,0, 6,0, 7,0, 8,0, 9,0 oder 10,0, insbesondere auf 7,0 eingestellt wird. Ein bevorzugtes Verfahren zur Darstellung der erfindungsgemäßen AGEs ist die Umsetzung mindestens eines Zuckers mit mindestens einer Aminosäure in einer wässrigen Pufferlösung. Das Volumen des Lösungsmittels wird anschließend reduziert und das Produktes isoliert, vorzugsweise durch Gefriertrocknung.
Erfindungsgemäße Lysin-basierte AGEs und/oder deren Vorläufer sind Produkte der Maillard-Reaktion von Lysin A mit Zuckern, nämlich Maltosin 15 sowie Pyridosin 15a, welche sich durch folgende Strukturformein auszeichnen:

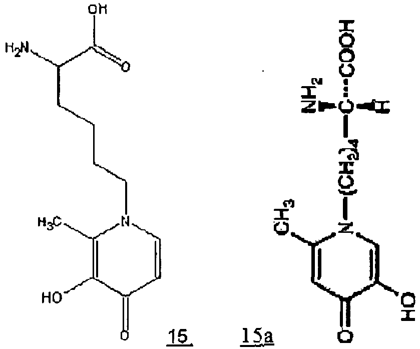

Eine bevorzugte Ausführungsform der Erfindung ist die Verwendung einer erfindungsgemäßen kosmetischen Zusammensetzung mit einem Anteil einer Verbindung oder einer Mischung verschiedener Verbindungen aus der Gruppe AGEs und/oder deren Vorläufern von 0,00001 bis 15 Gew.-%, vorzugsweise von 0,0001 bis 5 Gew.-%, besonders bevorzugt von 0,001 bis 1 Gew.-%, jeweils bezogen auf die Gesamtzusammensetzung.

Das kosmetische und pharmazeutische Wirkpotential der erfindungsgemäßen Gruppe AGEs (Advanced Glycation Endproducts) und/oder deren Vorläufern ist auf eine Reihe höchst vorteilhafter und überraschender Wirkeffekte dieser Substanzen zurückzuführen, die im Folgenden näher beschrieben werden.

Unter dem Begriff Hautzellen sind im Rahmen der vorliegenden Erfindung alle Zellen zu verstehen, die Bestandteil der menschlichen und/oder tierischen Haut selbst, der Schleimhaut sowie der Hautanhangsgebilde, sofern sie lebende Zellen umfassen, insbesondere der Haarfollikel, der Haarwurzel, der Haarzwiebel, des ventralen Epithels des Nagelbetts (Lectulus) sowie der Talg- und Schweißdrüsen sind.
Vorzugsweise sind unter dem Begriff Hautzellen auch organolypische Zellkulturen, wie sie beispielsweise als rekonstruierte dermale Papillen in der Europaischen Patentschrift 1455854 4beschrieben werden, zu verstehen.

Im Rahmen der vorliegenden Erfindung wurde weiterhin festgestellt, dass durch den Einsatz einer Zusammensetzung enthaltend mindestens eine Verbindung aus der Gruppe der AGEs und/oder deren Vorläufern ein Proliferations-hemmender Effekt erreicht werden kann (s. Beispiel 4). Substanzen, die einen solchen Effekt ausüben sind immer dann gewünscht, wenn die Zellteilung dysreguliert ist. Sie können beispielsweise, ohne sich darauf zu beschranken, in der Tumortherapie, gegen vermehrten Haarwuchs oder dickes Haar, bei gestörter epidermaler Homeostease oder einer übersteigerten Immunantwort eingesetzt werden.

Durch den Einsatz einer Zusammensetzung enthaltend mindestens eine Verbindung aus der Gruppe der AGEs und/oder deren Vorläufern konnte auch die Ausschüttung der Wachstumsfaktoren HGF (Hepatocyte Growth Factor) und KGF (Keratinocyte Growth Factor) in dermalen Papillenzellen reduziert werden (s. Beispiel 6).
Die beiden Wachstumsfaktoren HGF und KGF sind wichtige Regulatoren des Haarzyklus. Substanzen, die eine negative Regulation dieser Faktoren hervorrufen sind von großer Bedeutung, wenn eine Reduktion des Haarwuchses und/oder eine Reduktion der Haardicke gewünscht ist.

Eine Ausführungsform der Erfindung ist daher die Verwendung einer kosmetischen Zusammensetzung enthaltend mindestens eine Verbindung aus der Gruppe der AGEs und/oder deren Vorläufern, ausgewählt aus Verbindungen mit einer chemischen Struktur gemäß Formel 15 oder 15a, zur therapeutischen und nicht-therapeutischen Reduktion des Haarwuchses und/oder zur Reduktion der Haardicke, bevorzugt zur kosmetischen/nicht-therapeutischen Reduktion des Haarwuches und/oder Reduktion der Haardicke.

Gleichzeitig zeigen weiterführende biologische Tests, dass die erfindungsgemäßen AGEs und/oder deren Vorläufern in der Regel kelne zellschadlgenden Nebeneffekte, vorzugsweise keine membranschädigenden Effekte auf die Haut bzw. Hautzellen, vorzugsweise auf dermale Papillenzellen, ausüben (s. Beispiel 2). Vielmehr zeigen Untersuchungen zur Zellvitalität der Haut, vorzugsweise zur Zellvitalität kultivierter Fibroblasten, dass durch die Verwendung von Zusammensetzungen enthaltend mindestens eine Verbindung aus der Gruppe AGEs und/oder deren Vorläufern ein zellaktivierender Wirkstoffeffekt erzielt wird. Zellschädigende Substanzwirkungen werden in der Regel nicht beobachtet (s. Beispiel 1).

Eine weitere bevorzugte Ausführungsform ist die Verwendung einer erfindungsgemäßen kosmetischen Zusammensetzung, die mindestens eine weitere Komponente ausgewählt aus der Gruppe der Emulgatoren bzw. Tenside, kosmetischen Wirkstoffe, Vitamine, Proteinhydrolysate, filmbildende Substanzen, UV-Filtersubstanzen, Duftstoffe, Konditioniermittelwirkstoffe, Verdickungsmittel, Konservierungsmittel, Antioxidantien, Färbemittel, Puffermittel, Sequestrierungsprodukte, Treibmittel, Reduktionsmittel, Antischuppenwirkstoffe, Tanning-Mittel, Feuchthaltemittel der Haut und/oder Glanzmittel enthält.

Emulgatoren bewirken an der Phasengrenzfläche die Ausbildung von wasser- bzw. ölstabilen Adsorptionsschichten, die die dispergierten Tröpfchen gegen Koaleszenz schützen und damit die Emulsion stabilisieren. Emulgatoren sind daher wie Tenside aus einem hydrophoben und einem hydrophilen Molekolteil aufgebaut. Hydrophile Emulgatoren bilden bevorzugt O/W - Emulsionen und hydrophobe Emulgatoren bilden bevorzugt W/O - Emulsionen. W/O-Emulsionen, die ohne hydrophile Emulgatoren stabilisiert sind, sind in den Offenlegungsschriften DE 19816665 A1 und DE 19801593 A1 offenbart. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion.

Erfindungsgemäß verwendbare Emulgatoren sind beispielsweise
- Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₆-C₁₅-Alkylphenole,
- C₁₂-C₂₂-Fettsäuremono- und -diester von Anlagerungsprodukten von 1 bis 30 Mol Ethylenoxid an C₃-C₆-Polyole, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, z. B. das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten C₆-C₂₂-Fettsäuren,
- Sterole (Sterine). Als Sterole wird eine Gruppe von Steroiden verstanden, die am C-Atom 3 des Steroid-Gerustes eine Hydroxylgruppe tragen und sowohl aus tierischem Gewebe (Zoo-statole) wie auch aus pflanzlichen Fetten (Phytosterole) isoliert werden. Beispiele für Zoosterole sind das Cholesterol und das Lanosterol. Beispiele geeigneter Phytosterole sind Beta-Sitosterol, Stigmasterol, Campesterol und Ergosterol. Auch aus Pilzen und Hefen werden Sterole, die sogenannten Mykosterole, isoliert.
- Phospholipide, vor allem die Glucose-Phospolipide, die z. B. als Lecithine bzw. Phosphatidyl-choline aus z. B. Eidotter oder Pflanzensamen (z. B. Sojabohnen) gewonnen werden,
- Fettsäureester von Zuckern und Zuckeralkoholen wie Sorbit,
- Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI),
- Lineare und verzweigte C₆-C₃₀-Fettsäuren und deren Na-, K-, Ammonium-, Ca-, Mg- und Zn - Salze.

Die erfindungsgemäßen Mittel enthalten die Emulgatoren bevorzugt in Mengen von 0,1 bis 25 Gew.-%, insbesondere 0,5 - 15 Gew.-%, bezogen auf das gesamte Mittel.

In einer besonders bevorzugten Ausführungsform ist mindestens ein nichtionischer Emulgator mit einem HLB-Wert von 8 und darunter, gemäß den im Römpp-Lexikon Chemie (Eds.: J. Falbe, M. Regitz), 10. Auflage, Georg Thieme Verlag Stuttgart, New York, (1997), Seite 1764, aufgeführten Definitionen des HLB-Wertes, enthalten. Derart geeignete Emulgatoren sind beispielsweise Verbindungen der allgemeinen Formel R¹ - 0 - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen ist. Als Emulgator der Formel R¹ - O - R² besonders bevorzugt ist ein Behen- oder Erucylderivat, in welchem R¹ eine lineare, endständig substituierte Alkyl-, Alkenyl- oder Acylgruppe mit 22 C-Atomen darstellt.

Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensaure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythrit-monoerucat.

In einer anderen, ebenfalls besonders bevorzugten Ausführungsform ist mindestens ein ionischer Emulgator, ausgewählt aus anionischen, zwitterionischen, ampholytischen und kationischen Emulgatoren, enthalten. Bevorzugte anionische Emulgatoren sind Alkylsulfate, Alkylpolyglycol-ethersulfate und Ethercarbonsäuren mit 10 bis 18 C-Atomen in der Alkylgruppe und bis zu 12 Glycolathergruppen im Molekül, Sulfobernstelnsauremono- und -dialkylester mit 8 bis 18 C-Atomen in derAlkylgruppe und Sulfobernsteinsauremono-alkylpolyoxyethylester mit 8 bis 18 C-Atomen in der Alkylgruppe und 1 bis 6 Oxyethylgruppen, Monoglyceridsulfate, Alkyl- und Alkenyletherphosphate sowie Eiweißfettsäurekondensate. Zwitterionische Emulgatoren tragen im Molekül mindestens eine quartare Ammoniumgruppe und mindestens eine -COO⁻ - oder -SO₃⁻ -Gruppe. Besonders geeignete zwitterionische Emulgatoren sind die sogenannten Betaine wie die N-Alkyl-N,N-dimethylammonium-glycinate, N-Acyl-aminopropyl-N,N-dimethylammoniumglycinate und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.
Ampholytische Emulgatoren enthalten außer einer C₈-C₂₄-Alkyl- oder -Acylgruppe mindestens eine freie Aminogruppe und mindestens eine -COOH- oder -SO₃H-Gruppe im Molekül und können innere Salze ausbilden. Beispiele für geeignete ampholytische Emulgatoren sind N-Alkylglycine, N-Alkylaminopropionsäuren, N-Alkylaminobuttersäuren, N-Alkyliminodipropionsäuren, N-Hydroxy-ethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe.

Die ionischen Emulgatoren sind in einer Menge von 0,01 bis 5 Gew.-%, bevorzugt von 0,05 bis 3 Gew.-% und besonders bevorzugt von 0,1 bis 1 Gew.-%, bezogen auf das gesamte Mittel, enthalten.

Weiterhin können die erfindungsgemäßen Zusammensetzungen schäumende nichtionische, zwitterionische, anionische und kationische Tenside enthalten.
Beispiele für nichtionische Tenside sind
- alkoxylierte Fettsäurealkylester der Formel R¹CO-(OCH₂CHR²)ₓOR³,
   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und x für Zahlen von 1 bis 20 steht,
- Anlagerungsprodukte von Ethylenoxid an Fettsäureafkanotamide und Fettamine,
- Fettsäure-N-alkylglucamide,
- C₆ - C₂₂-Alkylamin-N-oxide,
- Alkylpolygykoside entsprechend der allgemeinen Formel RO-(Z)ₓ wobei R für eine C₆-C₁₆-Alkylgruppe, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeltung dieser Verbindungen vor. Besonders bevorzugt sind solche Alkylpolygfykoside, bei denen R im wesentlichen aus C₆- und C₁₀-Alkylgruppen, im wesentlichen aus C₁₂- und C₁₄-Alkylgruppen, im wesentlichen aus C₈. bis C₁₆-Alkylgruppen oder im wesentlichen aus C₁₂- bis C₁₆-Alkylgruppen besteht.
   Als Zuckerbaustein Z können beliebige Mono- oder Oligosaccharide eingesetzt werden. Üblicherweise werden Zucker mit 6 bzw. 6 Kohlenstoffatomen sowie die entsprechenden Oligosaccharide eingesetzt, beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt. Die erfindungsgemäß verwendbaren Alkylpolyglykoside enthalten im Schnitt 1,1 bis 5, bevorzugt 1,1 bis 2,0 besonders bevorzugt 1,1 bis 1,8 Zurkareinhaiten, Auch die alkoxylierten Homologen der genannten Alkylpolyglykoside können erfindungsgemaß eingesetzt werden. Diese Homologen können durchschnittlich bis zu 10 Ethylenoxid- und/oder Propylenoxideinheiten pro Alkylglykosideinheit enthalten.

Als zwltterlonische Tenside werden solche oberflächenaktiven Verbindungen bezeichnet, die im Molekül mindestens eine quartare Ammoniumgruppe und mindestens eine -COO⁽⁻⁾ - oder -SO₃⁽⁻⁾ - Gruppe tragen. Besonders geeignete zwitterionische Tenside sind die sogenannten Betaine wie die N-Alkyl-N,N-dimethylammoniumglycinate, beispielsweise das Kokosalkyldimethylammonium-glycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen In der Alkyl- oder Acylgruppe sowie das Kokosacylaminoethylhydroxyethylcarboxymethylglycinat. Ein bevorzugtes zwitterionisches Tensid ist das unter der INCl-Bezeichnung Cocamidopropyl Betaine bekannte Fettsäureamid-Derivat.

Als anionische Tenside eignen sich in erfindungsgemäßen Zubereitungen alle für die Verwendung am menschlichen Körper geeigneten anionischen oberffächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete schäumende Aniontenside sind, jeweils in Form der Nalrium-, Kalium- und Ammonium- sowie der Mono-, Di- und Trialkanolammoniumsalze mit 2 bis 4 Kohlenstoffatomen in der Alkanolgruppe, Acylglutamate der Formel (B-I), 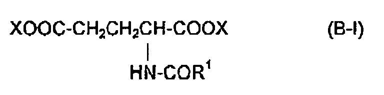 in der R'CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure. Palmitinsäure und/oder Stearinsäure, Insbesondere Natrium-N-cocoyl- und Natrium-N-stearoyl-L-glutamat,
- Ester einer hydroxysubstituierten Di- oder Tricarbonsäure der allgemeinen Formel (B-II), 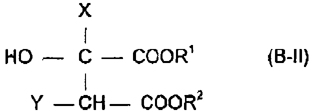 in der X=H oder eine -CH₂COOR-Gruppe ist, Y=H oder -OH ist unter der Bedingung, dass Y=H ist, wenn X=-CH₂COOR ist, R, R¹ und R² unabhängig voneinander ein Wasserstoffatom, ein Alkali- oder Erdalkalimetellkation, eine Ammoniumgruppe, das Kation einer ammonium-organischen Base oder einen Rest Z bedeuten, der von einer polyhydroxylierten organischen Verbindung stammt, die aus der Gruppe der veretherten(C₈-C₁₈)-Alkylpulysaccharide mit 1 bis 6 monomeren Saccharideinheiten und/oder der veretherten aliphatischen (C₈-C₁₈)-Hydroxyalkylpolyole mit 2 bis 16 Hydroxylresten ausgewählt sind, unter der Maßgabe, daß wenigstens eine der Gruppen R, R¹ oder R² ein Rest Z ist,
- Ester des Sulfobemsteinsäure-Salzes der allgemeinen Formel (B-III), 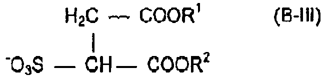 In der R¹ und R² unabhängig vonelnander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₈-C₁₈)-Alkylpolysaccharide mit 1 bis 6 monomeren Saccharideinheiten und/oder der veretherten aliphatischen(C₈-C₁₈)-Hydroxyalkylpolyole mil 2 bis 16 Hydroxylresten ausgewählt ist, unter der Maßgabe, daß wenigstens eine der Gruppen R¹ oder R² ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Ethoxygruppen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist.
- Acylsarcosinate mit einem linearen oder verzweigten Acylrest mit 6 bis 22 Kohlenstoffatomen und 0, 1, 2 oder 3 Doppelbindungen,
- Acyltaurate mit einem linearen oder verzweigten Acylrest mit 6 bis 22 Kohlenstoffatomen und 0, 1, 2 oder 3 Doppelbindungen,
- Acylisethionate mit einem linearen oder verzweigten Acylrest mit 6 bis 22 Kohlenstoffatomen und 0, 1, 2 oder 3 Doppelbindungen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)_{z}-SO₃X, in der R eine bevorzugt lineare Alkylgruppe mit 8 bis 30 C-Atomen, besonders bevorzugt mit 8 - 18 C-Atomen, z = 0 oder 1 bis 12, besonders bevorzugt 3, und X ein Natrium-, Kalium-, Magnesium-, Zink-, Ammoniumion oder ein Monoalkanol-, Dialkanol- oder Trialkanolammoniumion mit 2 bis 4 Kohlenstoffatomen in der Alkanolgruppe ist, wobei ein besonders bevorzugtes Beispiel Zinkcocoylethersulfat mit einem Ethoxylierungsgrad von z = 3 ist,
- Gemische oberflächenaktiver Hydroxysulfonate gemäß DE-A-37 25 030,
- sulfatierte Hydroxyalkylpolyethylen- und/oder Hydroxyalkylenpropylenglykotether gemäß DE-A-37 23 354,
- Sulfonate ungesättigter Fettsäuren mit 8 bis 24 C-Atomen und 1 bis 6 Doppelbindungen gemäß DE-A-39 26 344.
- Alkyl- und/oder Alkenyletherphosphate der Formel (B-IV) 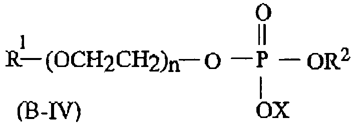
- in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für einen C₁ bis C₄ - Kohlenwasserstoffrest, steht,
- sulfatierte Fetlsäurealkylenglykolester der Formel R⁷CO(AlkO)ₙSO₃M , in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ Und/oder CH₂CHCH₃ n für Zahlen von 0,5 bis 6 und M für ein Kation steht, wie sie in der DE-OS 197 36 906.5 beschrieben sind,
- Monoglyceridsulfate und Monoglyceridethersulfate der Formel (B-V), 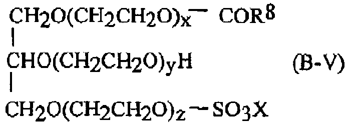
- in der R⁶CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali-oder Erdalkalimetall steht. Typische Beispiele für im Sinne der Erfindung geeignete Monoglycerld(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Taigfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (VI) eingesetzt, in der R⁸CO für einen linearen Acylrest mit 8 bis 18 Kohlenstoffatomen steht.

Der kosmetische Wirkstoff mit einem Anteil von 0,0001 - 38 Gew.-% bezogen auf die Gesamtmenge der Zusammensetzung wird bevorzugt ausgewählt aus folgenden Produkten oder deren Mischungen:
- schweißhemmenden Wirkstoffen
- desodorierenden Wirkstoffen
- Monomeren, Oligomeren und Polymeren von Aminosäuren, N-C₂-C₂₄-Acylaminosäuren, den Estern und/oder den physiologisch verträglichen Salzen dieser Substanzen
- DNA- oder RNA-Oligonucleotiden
- natürlichen Betainverbindungen
- α-Hydroxycarbonsäuren, α-Ketocarbonsäuren, β-Hydroxycarbonsäuren und deren Ester-, Lacton- oder Salzform,
- Flavonoiden und Flavonoid-reichen Pflanzenextrakten
- Isoflavonoiden und Isoflavonoid-reichen Pflanzenextrakten
- Polyphenolen und Polyphenol-reichen Pflanzenextrakten
- Ubichinon und Ubichinol sowie deren Derivaten
- Silymarin
- Ectoin
- Repellentien
- selbstbräunenden Wirkstoffen
- haulaufhellenden Wirkstoffen
- haulberuhlgenden Wirkstoffen
- feuchtigkeitsspendenden Wirkstoffen
- sebumregulierenden Wirkstoffen

In einer bevorzugten Ausführungsform enthalten die erfindungsgemäßen Zusammensetzungen mindestens ein Vitamin, Provitamin oder eine als Vitaminvorstufe bezeichnete Verbindung aus den Vitamingruppen A, B, C, E, H und K und den Estern der vorgenannten Substanzen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie Retinylpalmitat und Retinylacetat in Betracht. Die erfindungsgemäßen Zusammensetzungenen enthalten die Vitamin A-Komponente bevorzugt In Mengen von 0,05 - 1 Gew.-%, bezogen auf die gesamte Zusammensetzung.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid Bevorzugt wird Thiaminhydrochlorid in Mengen von 0,05 bis 1 Gew.%, bezogen auf das gesamte Mittel, eingesetzt.
- Vitamin B₂, Trivialname Riboflavin, chemische Bezeichung 7,8-Dimethyl-10-(1-D-ribityl)-benzo[g]pteridin-2,4(3H,10H)-dion. Bevorzugt werden Riboflavin oder seine Derivate in Mengen von 0,05 bis 1 Ges.-%, bezogen auf das gesamte Mittel, eingesetzt
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0.05 bis 1 Gew.-%, bezogen auf das gesamte Mittel, enthalten ist. 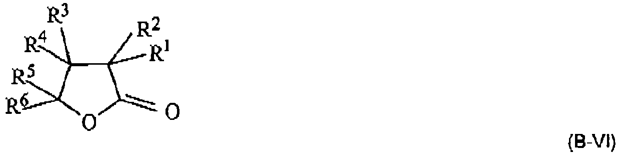
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ Ist In den erfindungsgemäßen Mitteln bevorzugt in Mengen von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3aS,4S, 6aR)-2-Oxohexahydrothienol[3,4-d]-imidazol-4-valeriansäure. Biotin ist in den erfindungsgemäßen Mitteln bevorzugt In Mengen von 0,0001 bis 1.0 Gew.-%, insbesondere in Mengen von 0,001 bis 0,01 Gew.-% enthalten.

Vitamin C (Ascorbinsäure) wird bevorzugt in Mengen von 0,1 bis 3 Gew.%, bezogen auf die gesamte Zusammensetzung, eingesetzt. Die Verwendung der Derivate Ascorbylpalmitat, -stearat,-dipalmitat, -acetat, Mg-Ascorbylphosphat, Na-Ascorbylphosphat. Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein.

Zur Vitamin E-Gruppe zählen Tocopherol, Insbesondere α-Tocopherol, und seine Derivate. Bevorzugte Derivate sind insbesondere die Ester, wie Tocopherylacetat, -nicotinat, -phosphat,-succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.%, bezogen auf die gesamte Zusammensetzung, enthalten.
Unter Vitamin F werden üblicherweise essentielle Fettsäuren, insbesondere Linolsäure, Linolensäure und Arachidonsäure, verstanden.

Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Famochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweits bezogen auf die gesamte Zusammensetzung, enthalten.
Vitamin A-palmitat (Retinylpalmitat), Penthenol, , Nicotinsäureamid, Pyridoxin, Pyrldoxamin, Pyridoxal, Biotin, Ascorbylpalmilat, -acetat, Mg-Ascorbylphosphat, Na-Ascorbylphosphat, Natrium- und Magneslumascorbat und die Tocopherolester, besonders Tocopherylacetat, sind erfindungsgemäß besonders bevorzugt.

Vorzugsweise findet eine kosmetischen Zusammensetzung Verwendung, die in Form einer Salbe, Tablette, Lotion, Creme, Emulsion, Suspension, Milch, Paste, Gel, Schaum oder Spray, insbesondere in emulgierter Form vorliegt.

### Ausführungsbeispiele

### Beispiel 1

### Nachweis der Freisetzung von Wachstumsfaktoren

Die beiden Wachstumsfaktoren Hepatocyte Growth Factor (HGF) und Keratinocyte Growth Factor (KGF) sind wichtige Regulatoren des Haarzyklus. Bei den Haarzyklus regulierenden Substanzen sollte eine Veränderung in der Verfügbarkeit im Vergleich zur nicht behandelten Kontrolle ausgegangen werden. Die Ausschüttung von HGF sowie KGF kann mit Hilfe von kommerziell erhältlichen ELISA-Kits (Quantikine, RnD-Systems, Wiesbaden, Deutschland) quantifiziert werden. Dazu werden rekonstruierte Haarfollikelmodelle, die in der Europäischen Patentschrift1455854 beschrieben sind, über 72h mit Testsubstanzen inkubiert und die Konzentration der Wachstumsfaktoren im Medium in beschriebener Weise bestimmt. Um ausschließen zu können, dass die beschriebenen Effekte durch nicht umgesetzte Zucker (Glucose oder Lactose) zu Stande gekommen sind, wurden die jeweiligen Einzelkomponenten in der höchsten möglichen Konzentration getestet. Die Quantitäten der unbehandelten Kontrollen werden gleich 100 % gesetzt und die Quantitäten der behandelten Proben darauf bezogen. Die Standardabweichungen errechnen sich aus den drei biologischen Ansätzen.

Beide Wachstumsfaktoren sind wichtige Boten für die Regulation des Haarzyklus. HGF und KGF werden von den dermalen Papillenzellen des Haarfollikels produziert und freigesetzt, um vor allem in der Wachstumsphase (Anagen) Prozesse wie die Zellteilungsaktivität der Haarkeratinozyten zu steuern. Werden, wie für einige Substanzen beschrieben diese Wachstumsfaktoren vermindert freigesetzt, kann das zu einem potentiellen Stop der Wachstumsphase führen. Dies bedeutet, dass das Haar nicht mehr wächst, in die Regressionsphase des Haarzyklus übergeht und ausfällt.

Dies zugrunde legend besitzen die getesteten Substanzen Potential als Anti-Haarwuchsaktivstoff.

### Beispiel 2:

Weiterhin wurde der Einfluss von AGEs auf das Genexpressionsprofil von dermalen Papillenzellen getestet.
Dazu wurden rekonstruierte dermale Papillen, wie sie in der Europäischen Patentschrift1455854 beschrieben sind, über 24 h mit den jeweiligen in der Tabelle 1 angegebenen Testsubstanzen in den dort angegebenen Konzentrationen systemisch behandelt. Danach wurde das relative Genexpressionsprofil für die Marker HGF und KGF (wichtige Marker der anagenen Phase des Haarzyklus) und TGF-beta 1, TGFbela2 und IGFBP3 (wichtige Marker der katagenen Phase des Haarzyklus) bestimmt. Als Referenz diente die Expression bei Inkubation mit unbehandeltem Medium, die in der Tabelle angegebenen Werte stellen die differenzielle Genexpression dar. Eine differenzielle Genexpression, die das 1,66 fache oder größere absolute Zahlenwerte der Kontroll-Genexpression beträgt, ist als signifikant anzusehen.

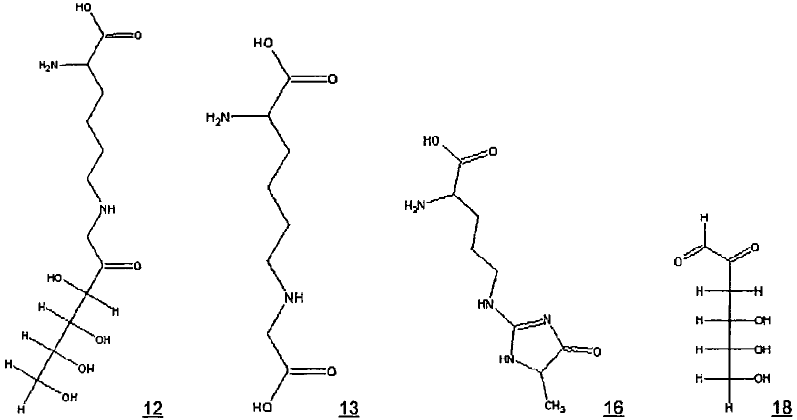

**Tabelle 1: Untersuchung des relativen Genexpressionsprofils relevanter Marker des Haarzyklus bei Inkubation mit verschiedenen AGEs und/oder deren Vorläufer, Angabe der Einsatz-Konzentrationen in Gew.-%; Die in Klammern hinter die Substanznamen gesetzten Zahlen verweisen auf die oben angegebenen Strukturformeln.**

| | | **Anagen** | | **Katagen** | | |
|---|---|---|---|---|---|---|
| **Substanzen** | **Konz.** | **HGF** | **KGF** | **TGFß1** | **TGFß2** | **IGFBP3** |
| unbehandelt / nur Medium | | 1,00 | 1,00 | 1,00 | 1,00 | 1,00 |
| Maltol | 0,025% | 1,07 | **-3,02** | -1,20 | **-3,21** | **-3,63** |
| Maltol | 0,01% | -1,23 | **-1,87** | -1,28 | **-1,86** | -1,69 |
| Fructoselysin (12) | 0,1% | -1,69 | **-2,49** | -1,10 | **-2,40** | **-1,83** |
| Fructoselysin | 0,01% | **-1,92** | **-1,96** | 1,04 | **-2,40** | -1,44 |
| 3-Desoxyglucosulose (18) | 0,1% | **-1,78** | **-1,93** | -1,27 | -1,54 | **-2,12** |
| 3-Desoxyglucosulose | 0,01% | **-2,09** | -1,33 | 1,02 | -1,67 | -1,20 |
| Omithinoimidazolinon (16) | 0,1% | **-3,30** | -1,25 | 1,08 | **-1,66** | -1,60 |
| Maltosin (15) | 0,1% | **-2,09** | -1,45 | **3,39** | **-2,24** | **7,55** |
| Carboxymethyllysin (13) | 0,1% | -1,77 | **1,24** | 1,35 | -1,07 | **1,98** |

Die Reduktion der Genexpression von Markern des Anagens und damit eine reduzierte Ausschüttung der Wachstumsfaktoren ist, wie bereits unter Beispiel 1 erläutert, ein deutlicher Hinweis auf die Eignung der getesteten Substanzen (gemäß Tabelle 1) zur Verwendung als Anti-Haarwuchsmittel (insbesondere kosmetische/bzw. nicht-therapeutische Verwendung).

Eine dazu gleichzeitig erhöhte Genexpression von Markern des Katagen ist insbesondere bei 0,1 Gew.-% Maltosin zu beobachten. Die TGF-beta 1 induziert die Apoptose (den programmierten Zelltod) in Keratinozyten und beendet somit die Elongation des Haarschaftes und damit das Haarwachstum. Maltosin erscheint besonders geeignet zur Verwendung als Anti-Haarwuchsmittel (insbesondere im Rahmen einer kosmetischen bzw. nicht-therapeutischen Verwendung).

## Patentansprüche

1. Maltosin zur Verwendung als Therapeutisches Anti-Haarwuchsmittel.

2. Nicht-therapeutische Verwendung von Maltosin als Anti-Haarwuchsmittel.

3. Verwendung von mindestens einer Verbindung aus der Gruppe der Advanced Glycation Endproducts (AGEs) und/oder deren Vorläufern, ausgewählt aus Verbindungen mit einer chemischen Struktur gemäß Formel 15 oder 15a, zur Herstellung eines therapeutischen Hautbehandlungsmittels zur Reduktion des Haarwuchses.

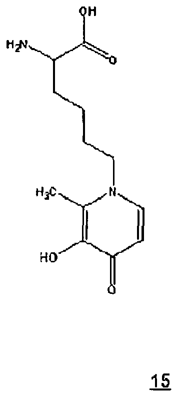

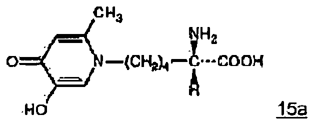

4. Nicht-therapeutische Verwendung einer kosmetischen Zusammensetzung, enthaltend mindestens eine Verbindung aus der Gruppe der Advanced Glycation Endproducts (AGEs) und/oder deren Vorläufern, ausgewählt aus Verbindungen mit einer chemischen Struktur gemäß Formel 15 oder 15a, als Hautbehandlungsmittel zur Reduktion des Haarwuchses und/oder zur Reduktion der Haardicke.

5. Verwendung nach mindestens einem der Ansprüche 2, 3 oder 4, **dadurch gekennzeichnet, dass** der Anteil einer Verbindung oder einer Mischung verschiedener Verbindungen aus der Gruppe Advanced Glycation Endproducts (AGEs) und/oder deren Vorläufern 0,00001 bis 15 Gew.-%, vorzugsweise 0,0001 bis 5 Gew.-%, besonders bevorzugt 0,001 bis 1 Gew.-% In der Gesamtzusammensetzung beträgt.

6. Verwendung nach mindestens einem der Ansprüche 2 bis 5, **dadurch gekennzeichnet, dass** das Hautbehandlungsmittel in Form einer Salbe, Tablette, Lotion, Creme, Emulsion, Suspension, Milch, Paste, eines Gels, Schaums oder Sprays, insbesondere in emulgierter Form, vorliegl.

7. Nicht-therapeutische Verwendung einer kosmetischen Zusammensetzung, enthaltend mindestens eine Verbindung aus der Gruppe der Lysin-basierten AGEs und/oder deren Vorläufern, ausgewählt aus Verbindungen mit einer chemischen Struktur gemäß Formel 15 oder 15a, zur Hautbehandlung.

8. Nicht-therapeutische Verwendung gemäß Anspruch 7, **dadurch gekennzeichnet, dass** die kosmetische Zusammensetzung in Form einer Salbe, Tablette, Lotion, Creme, Emulsion, Suspension, Milch, Paste, eines Gels, Schaums oder Sprays, insbesondere in emulgierter Form, vorliegt.

9. Nicht-therapeutische Verwendung gemäß Anspruch 7, **dadurch gekennzeichnet, dass** die kosmetische Zusammensetzung mindestens eine weitere Komponente, ausgewählt aus der Gruppe der Emulgatoren, Tenside, kosmetischen Wirkstoffe, Vitamine, Proteinhydrolysate, filmbildenden Substanzen, UV-Filtersubstanzen, Duftstoffe, Konditioniermittelwirkstoffe, Verdickungsmittel, Konservierungsmittel, Antioxidantien, Färbemittel, Puffermittel, Sequestrierungsprodukte, Treibmittel, Reduktionsmittel, Antischuppenwirkstoffe, Tanning-Mittel, Feuchthaltemittel der Haut und/ oder Glanzmittel, enthält.

## Claims

1. Maltosine for use as a therapeutic anti-hair growth agent.

2. Non-therapeutic use of maltosine as an anti-hair growth agent.

3. Use of at least one compound from the group of the advanced glycationend products (AGEs) and/or of their precursors, selected from compounds with a chemical structure according to Formula 15 or 15a, for the preparation of a therapeutic skin-treatment agent for reducing hair growth.

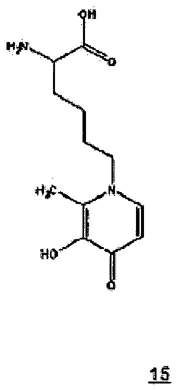

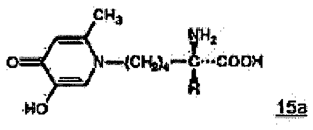

4. Non-therapeutic use of a cosmetic composition, comprising at least one compound from the group of the advanced glycation end products (AGEs) and/or of their precursors, selected from compounds with a chemical structure according to Formula 15 or 15a, as a skin-treatment agent for reducing hair growth and/or for reducing hair thickness.

5. The use according to at least one of claims 2, 3 or 4, **characterised in that** the fraction of a compound or a mixture of different compounds from the group of advanced glycation end products (AGEs) and/or their precursors is 0.00001 to 15 wt %, preferably 0.0001 to 5 wt %, particularly preferably 0.001 to 1 wt % in the total composition.

6. The use according to at least one of claims 2 to 5, **characterised in that** the skin-treatment agent is in the form of a balm, tablet, lotion, cream, emulsion, suspension, milk, paste, a gel, foam or spray, particularly in emulsified form.

7. Non-therapeutic use of a cosmetic composition, comprising at least one compound from the group of the lysine-based AGEs and/or of their precursors, selected from compounds with a chemical structure according to Formula 15 or 15a, for skin-treatment.

8. The non-therapeutic use according to claim 7, **characterised in that** the cosmetic composition is in the form of a balm, tablet, lotion, cream, emulsion, suspension, milk, paste, a gel, foam or spray, particularly in emulsified form.

9. The non-therapeutic use according to claim 7, **characterised in that** the cosmetic composition comprises at least one additional component, selected from the group of the emulsifiers, surfactants, cosmetic active ingredients, vitamins, protein hydrolysates, film-forming substances, UV-filter substances, fragrances, conditioning agent active ingredients, thickeners, preservatives, antioxidants, colorants, buffers, sequestrants, propellants, reducing agents, anti-dandruff active ingredients, tanning agents, moisturisers for the skin and/or lustering agents.

## Revendications

1. Maltosine pour son utilisation à titre d'agent thérapeutique s'opposant à la pousse des poils.

2. Utilisation non thérapeutique de maltosine à titre d'agent s'opposant à la pousse des poils.

3. Utilisation d'au moins un composé du groupe des Advanced Glycation Endproducts (AGE) et/ou de leurs précurseurs, choisi parmi des composés dont la structure chimique répond à la formule 15 ou 15a pour la préparation d'un agent thérapeutique de traitement cutané pour la réduction de la pousse des poils.

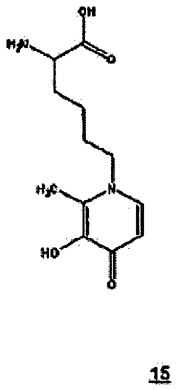

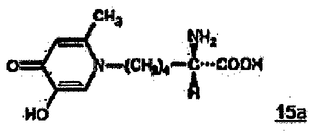

4. Utilisation non thérapeutique d'une composition cosmétique, contenant au moins un composé du groupe des Advanced Glycation Endproducts (AGE) et/ou de leurs précurseurs, choisi parmi des composés dont la structure chimique répond à la formule 15 ou 15a comme agent de traitement cutané pour la réduction de la pousse des poils et/ou pour la réduction de l'épaisseur des poils.

5. Utilisation selon au moins une des revendications 2, 3 ou 4, **caractérisée en ce que** la fraction d'un composé ou d'un mélange de différents composés du groupe des Advanced Glycation Endproducts (AGE) et/ou de leurs précurseurs s'élève de 0,00001 à 15 % en poids, de préférence de 0,0001 à 5 % en poids, de manière particulièrement préférée de 0,001 à 1 % en poids dans la composition totale.

6. Utilisation selon au moins une des revendications 2 à 5, **caractérisée en ce que** l'agent de traitement cutané est présent sous la forme d'une pommade, d'un comprimé, d'une lotion, d'une crème, d'une émulsion, d'une suspension, d'un lait, d'une pâte, d'un gel, d'une mousse ou d'un spray, en particulier sous une forme émulsifiée.

7. Utilisation non thérapeutique d'une composition cosmétique contenant au moins un composé du groupe des AGE à base de lysine et/ou de leurs précurseurs, choisi parmi des composés dont la structure chimique répond à la formule 15 ou 15a pour le traitement de la peau.

8. Utilisation non thérapeutique selon la revendication 7, **caractérisée en ce que** la composition cosmétique est présente sous la forme d'une pommade, d'un comprimé, d'une lotion, d'une crème, d'une émulsion, d'une suspension, d'un lait, d'une pâte, d'un gel, d'une mousse ou d'un spray, en particulier sous une forme émulsifiée.

9. Utilisation non thérapeutique selon la revendication 7, **caractérisée en ce que** la composition cosmétique contient au moins un composant supplémentaire choisi parmi le groupe des émulsifiants, des agents tensioactifs, des substances actives cosmétiques, des vitamines, des hydrolysats de protéines, des substances filmogènes, des substances faisant office de filtre pour le rayonnement ultraviolet, des substances odoriférantes, des substances actives faisant office de revitalisants capillaires, des épaississants, des conservateurs, des antioxydants, des colorants, des agents faisant tampon, des produits de séquestration, des agents propulseurs, des agents de réduction, des substances actives antipelliculaires, des agents bronzants, des agents qui maintiennent l'humidité de la peau et/ou des agents conférant du brillant.
